# EUROPÄISCHE PATENTSCHRIFT

(11) **EP 0 879 274 B1**
(45) Veröffentlichungstag und Bekanntmachung des Hinweises auf die Patenterteilung: **16.05.2001**
(21) Anmeldenummer: 97902282.9
(22) Anmeldetag: 01.02.1997
(51) Int. Cl.: C11C 3/04, C07C 67/03, C07C 69/30, A61K 7/00, A61K 31/00

(54) **TECHNISCHE DI-/TRIGLYCERIDGEMISCHE**
TECHNICAL DI- AND TRIGLYCERIDE MIXTURES
MELANGES DE DI/TRIGLYCERIDES TECHNIQUES

(30) Priorität: 09.02.1996 DE 19604744
(43) Veröffentlichungstag der Anmeldung: 25.11.1998
(73) Patentinhaber: Cognis Deutschland GmbH, 40589 Düsseldorf (DE)
(72) Erfinder: PODUBRIN, Stefan, D-45481 Mülheim an der Ruhr (DE); ANSMANN, Achim, D-40699 Erkrath (DE); KAWA, Rolf, D-40789 Monheim (DE); RIDINGER, Richard, D-40789 Monheim (DE)
(86) Internationale Anmeldenummer: EP9700434
(87) Internationale Veröffentlichungsnummer: WO9729170

(56) Entgegenhaltungen:
- DE-A- 2 711 470
- GB-A- 160 840
- GB-A- 808 634
- GB-A- 820 269
- GB-A- 820 270
- US-A- 5 254 331
- DATABASE WPI Section Ch, Week 9402 Derwent Publications Ltd., London, GB; Class D14, AN 94-012595 XP002033370 & JP 05 320 677 A (NIPPON OIL KK) , 3.Dezember 1993
- DATABASE WPI Section Ch, Week 9251 Derwent Publications Ltd., London, GB; Class D13, AN 92-419615 XP002033371 & JP 04 314 790 A (NIPPON OIL KK) , 5.November 1992
- DATABASE WPI Section Ch, Week 8751 Derwent Publications Ltd., London, GB; Class B04, AN 87-359752 XP002033372 & JP 62 263 143 A (KAO CORP) , 16.November 1987

## Beschreibung

### Gebiet der Erfindung

Die Erfindung betrifft die Verwendung von technische Di-/Triglyceridgemischen als Ölkörper zur Herstellung von kosmetischen und/oder pharmazeutischen Zubereitungen, die man durch Umesterung von Pflanzenölen mit Methylestern erhält, sowie ein Verfahren zu ihrer Herstellung

### Stand der Technik

Zur Herstellung von kosmetischen bzw. pharmazeutischen Zubereitungen bedarf es in der Regel einer hydrophilen und einer hydrophoben Phase. Für die Formulierung derartiger Mittel kann der Fachmann auf eine umfangreiche Palette von Ölkörpern zurückgreifen. Aus Kostengründen werden jedoch vielfach preiswerte Paraffinölfraktionen eingesetzt, die zwar ein vorteilhaftes Viskositätsverhalten aufweisen und sich auch zum Lösen vieler Wirkstoffe eignen, jedoch nicht in das vom Markt gewünschte Konzept einer "grünen", d.h. überwiegend auf natürlichen, vorzugsweise pflanzlichen Rohstoffen basierenden Formulierung passen. Als Alternativen kommen selbstverständlich Rohstoffe wie beispielsweise Glycerintricaprylat in Betracht, die jedoch durch gezielte und technisch aufwendige Synthese aus Glycerin und Caprylsäure hergestellt werden müssen und daher etwa dreimal so teuer wie Paraffinölfraktionen sind. Die Patentschrift **GB 820270A 1** offenbart ein Verfahren zur Herstellung von Glyceriden, bei dem man mittelkettige Pflanzenöle mit kurzkettigen Triglyceriden umestert. Gegenstand der **WPI AN 94/012595** und **WPI** AN **92/419615** sind Di-/Triglyceridgemische, die durch partielle Umesterung von Kokos- und Palmenöl mit Methylestern erhalten werden.

Die komplexe Aufgabe der Erfindung hat somit darin bestanden, neue Ölkörper zur Verfügung zu stellen, die sich aus pflanzlichen Rohstoffen mit einem Minimum an Aufwand herstellen lassen und daher eine ökonomisch vertretbare Alternative zu Paraffinölfraktionen darstellen. Gleichzeitig sollten die Ölkörper eine komplexes Anforderungsprofil erfüllen, nämlich hellfarbig und geruchsfrei sein, eine ausreichende Lager- und Kältestabilität besitzen und insbesondere einen Trübungspunkt unterhalb von +6°C aufweisen.

### Beschreibung der Erfindung

Gegenstand der Erfindung ist die Verwendung von technischen Di-/Triglyceridgemischen, dadurch erhältlich, daß man raffinierte, überwiegend gesättigte Pflanzenöle mit (a) einer Mischung aus Glycerin und Fettsäuren der Formel (I)

**R**^{**1**}**COOH** **(I)**

bzw. deren Methylestern oder (b) Triglyceriden auf Basis der Fettsäuren der Formel **(I)**, in der R¹CO für einen Acylrest mit 6 bis 10 Kohlenstoffatomen steht, umestert, d.h. einen Teil der längerkettigen Fettsäuren des Pflanzenöls gegen kürzerkettige Fettsäuren austauscht, als Ölkörper in kosmetischen Zubereitungen.

Überraschenderweise wurde gefunden, daß sich durch partielle Umesterung von einfachen raffinierten Pflanzenölen mit kurzkettigen Fettstoffen der Titer soweit herabsetzen läßt, daß die Produkte nicht nur bei Raumtemperatur flüssig werden, sondern auch die geforderte Lager- und Kältestabilität aufweisen.

Ein weiterer Gegenstand der Erfindung betrifft ein Verfahren zur Herstellung von technischen Di-/Triglyceridgemischen, bei dem (a) einer Mischung aus Glycerin und Fettsäuren der Formel **(I)**

**R**^{**1**}**COOH** **(I)**

bzw. deren Methylestern oder (b) Triglyceriden auf Basis der Fettsäuren der Formel **(I)**, in der R¹CO für einen Acylrest mit 6 bis 10 Kohlenstoffatomen steht, umestert.

### Pflanzenöle

Als Einsatzstoffe kommen überwiegend gesättigte Pflanzenöle in Betracht, die eine lodzahl im Bereich von 0,5 bis 50 aufweisen. Die Pflanzenöle leiten sich von Fettsäuren ab, die 6 bis 22 Kohlenstoffatomen aufweisen können, wobei jedoch der Schwerpunkt der C-Kettenverteilung im Bereich von 12 bis 18 Kohlenstoffatomen liegt. Dies bedeutet, daß mindestens 80 % der in den Pflanzenölen enthaltenen Fettsäuren 12 bis 22 und vorzugsweise 12 bis 18 Kohlenstoffatome aufweisen. Typische Beispiele sind Palmöl, Palmkernöl, Babassuöl und/oder Kokosöl, deren Einsatz auch besonders bevorzugt ist. Pflanzenöle, die nach Härtung, d.h. Absenkung der lodzahl, in Betracht kommen, sind Olivenöl, Sonnenblumenöl, Rapsöl, Erdnußöl, Baumwollsaatöl, Teesamenöl, Chaulmoograöl, Korianderöl, Leinöl und Meadowfoamöl.

### Fettsäuren, Fettsäuremethylester und Triglyceride

Für die partielle Umesterung in Gegenwart von Glycerin geeignete Fettsäuren sind Capronsäurem Caprinsäure und insbesondere Caprylsäure. Anstelle der Fettsäuren können auch die entsprechenden Methylester eingesetzt werden. In einer Variante der Erfindung kann man die partielle Umesterung auch unmittelbar mit Triglyceriden auf Basis der genannten Fettsäuren durchführen; die Mitverwendung von Glycerin kann dann entfallen.

### Umesterung

Bei der Reaktion der Pflanzenöle mit den oben genannten Fettstoffen und gegebenenfalls dem Glycerin laufen verschiedene Reaktionen nebeneinander ab, die zu einer komplexen Mischung von Di- und Triglyceriden führen. So findet beispielsweise im Fall der Methylester eine partielle Umesterung der Pflanzenöle statt, d.h. zumindest ein Teil der längerkettigen Fettsäuren des Pflanzenöls wird gegen die kürzerkettigen Fettsäuren des Methylesters ausgetauscht. Die freigesetzten längerkettigen Fettsäuren können dann ihrerseits mit dem freien Glycerin Ester bilden. Ebenfalls möglich ist unter diesen Bedingungen eine Umesterung der kurzkettigen Methylestern mit dem Glycerin.

Die komplexe Reaktion, die hier der Einfachheit halber als "partielle Umesterung" bezeichnet wird, läuft üblicherweise bei Temperaturen im Bereich von 140 bis 250 und vorzugsweise 210 bis 230°C ab. Als Katalysatoren eignen sich für diesen Zweck bekannte Stoffe wie beispielsweise Zinkseifen, Zinnschliff, Zinnoxide, Titansäureester, Alkalihydroxide, -carbonate bzw. -alkoholate und dergleichen, die in Mengen von 0,05 bis 1 und vorzugsweise 0,1 bis 0,5 Gew.-% - bezogen auf die Einsatzstoffe - eingesetzt werden. Es empfiehlt sich, das bei der Umesterung freigesetzte Methanol kontinuierlich aus dem Reaktionsgleichgewicht zu entfernen und den Katalysator nach Abschluß der Reaktion beispielsweise durch Zugabe von Bleicherde zu neutralisieren, um im Verlauf der destillativen Abtrennung nichtabreagierten Methylesters keine Rückreaktionen zu katalysieren.

Es hat sich ferner als vorteilhaft erwiesen, die Pflanzenöle, die Fettsäuren bzw. Methylester und das Glycerin im molaren Verhältnis 1 : (2,5 bis 3,5) : (1,0 bis 2,0) einzusetzen, wobei molare Verhältnisse von 1 : (3,0 bis 3,4) : (1,3 bis 1,6) besonders bevorzugt sind, weil unter diesen Bedingungen ein praktisch vollständiger Umsatz resultiert. Werden Triglyceride eingesetzt, ergibt sich das Verhältnis in gleicher Weise, muß aber natürlich auf die Zahl der Acylgruppen berechnet werden. Wie schon erwähnt, ist in diesen Fällen die Mitverwendung von Glycerin nicht erforderlich, wenngleich in geringen Mengen möglich. Es werden in allen Fällen Produkte erhalten, die einen Monoglyceridanteil unterhalb von 5 Gew.-% aufweisen und bei denen das Gewichtsverhältnis zwischen Di- und Triglyceriden im Bereich von 1 : 3 bis 1 : 6 liegt. Derartige Di-/Triglyceridgemische zeichnen sich durch optimale anwendungstechnische Eigenschaften aus.

Falls erforderlich, können die nach der Umesterung erhaltenen Di-/Triglyceridgemische noch in an sich bekannter Weise einer Desodorierung unterworfen werden. Hierbei werden die Öle üblicherweise kontinuierlich in einer Fallstromkolonne oder diskontinuierlich in einem Kessel mit heißem Wasserdampf behandelt, wodurch die wasserdampfflüchtigen Geruchsträger (z.B. kurzkettige Aldehyde oder Ketone) praktisch quantitativ entfernt werden.

### Gewerbliche Anwendbarkeit

Die erfindungsgemäßen technischen Di-/Triglyceridgemische sind bei Raumtemperatur flüssig, weisen einen Kältetrübungspunkt unterhalb von +6°C aus, sind hellfarbig, geruchsfrei und lagerstabil. Ein weiterer Gegenstand der Erfindung betrifft daher ihre Verwendung als Ölkörper zur Herstellung von kosmetischen und/oder pharmazeutischen Zubereitungen, in denen sie in Mengen von 1 bis 95 und vorzugsweise 15 bis 50 Gew.-% enthalten sein können.

Typische Beispiele für Zubereitungen, in denen die neuen Ölkörper eingesetzt werden können, sind Hautpflegemittel wie beispielsweise Tagescremes, Nachtcremes, Pflegecremes, Nährcremes, Bodylotions, Sonnenschutzpräparaten, Salben und dergleichen, die als weitere Hilfs- und Zusatzstoffe zusätzliche Ölkörper, Tenside, Emulgatoren, Überfettungsmittel, Stabilisatoren, Wachse, Konsistenzgeber, Verdickungsmittel, Kation-polymere, Siliconverbindungen, biogene Wirkstoffe, Antischuppenmittel, Filmbildner, Konservierungs-mittel, Hydrotrope, Solubilisatoren, UV-Adsorber, Farb- und Duftstoffe enthalten.

Als zusätzliche **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, Guerbetcarbonate, Dialkylether, Siliconöle und/oder aliphatische bzw. naphthenische Kohlenwasserstoffe in Betracht.

Typische Beispiele für geeignete **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate.

Als **Emulgatoren** kommen beispielsweise nichtionogene Tenside aus mindestens einer der folgenden Gruppen in Frage:
- (b1): Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- (b2): C_{12/18}-Fettsäuremono- und -diester von Anlagerungsprodukten von 1 bis 30 Mol Ethylenoxid an Glycerin;
- (b3): Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- (b4): Alkylmono- und -oligoglycoside mit 8 bis 22 Kohlenstoffatomen im Alkylrest und deren ethoxylierte Analoga;
- (b5): Anlagerungsprodukte von 15 bis 60 Mol Ethylenoxid an Ricinusöl und/oder gehärtetes Ricinusöl;
- (b6): Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
- (b7): Anlagerungsprodukte von 2 bis 15 Mol Ethylenoxid an Ricinusöl und/oder gehärtetes Ricinusöl;
- (b8): Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
- (b9): Trialkylphosphate;
- (b10): Wollwachsalkohole;
- (b11): Polysiloxan-Polyalkyl-Polyether-Copolymere bzw. entsprechende Derivate;
- (b12): Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
- (b13): Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Saize, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quatemierte VinylpyrrolidonNinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure, Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### Beispiel 1:

In einer 2-l-Destillationsapparatur wurde ein Gemisch aus 657g (1 mol) raffiniertem Kokosöl, 665g (4,2 mol) Caprylsäuremethylester, 129g (1,4 mol) Glycerin und 1,5g Zinn(II)oxid - entsprechend 0,1 Gew.-% bezogen auf die Einsatzstoffe - unter Rückfluß 8h auf 230°C erhitzt und das freigesetzte Methanol kontinuierlich aus dem Reaktionsgleichgewicht entfernt. Danach wurde der nichtumgesetzte Methylester abdestilliert und der Katalysator durch Zugabe von 2g Bleicherde neutralisiert. Abschließend wurde das resultierende Öl durch eine Behandlung mit heißem Wasserdampf desodoriert. Es wurden 1100g eines leicht gelb gefärbten, geruchfreien Öls erhalten, das 0,8 Gew.-% Monoglyceride, 22,0 Gew.-% Diglyceride und 77,2 Gew.-% -Triglyceride enthielt.

### Beispiel 2:

Analog Beispiel 1 wurden 673g (1 mol) raffiniertes Palmkernöl, 679g (4,3 mol) Caprylsäuremethylester, 132g (1,4 mol) Glycerin und 1,5g Zinn(II)oxid - entsprechend 0,1 Gew.-% bezogen auf die Einsatzstoffe - unter Rückfluß 6h auf 220°C erhitzt. Es wurden 1080g eines leicht gelb gefärbten, geruchfreien Öls erhalten, das 2,2 Gew.-% Monoglyceride, 20,2 Gew.-% Diglyceride und 75,9 Gew.-% -Triglyceride enthielt.

### Beispiel 3:

Analog Beispiel 1 wurden 657g (1 mol) raffiniertes Kokosöl, 499g (3,2 mol) Vorlauffettsäure (C-Kette: C₆₋₁₀), 129g (1,4 mol) Glycerin und 1,5g Zinn(II)oxid - entsprechend 0,1 Gew.-% bezogen auf die Einsatzstoffe - 6h auf 220°C unter kontinuierlichem Entfernen des Reaktionswassers erhitzt. Es wurden nach Raffination und Desodorieren 1070g eines leicht gelb gefärbten, geruchfreien Öls erhalten, das 1,8 Gew.-% Monoglyceride, 20,2 Gew.-% Diglyceride und 78,0 Gew.-% Triglyceride enthielt.

### Beispiel 4:

Analog Beispiel 1 wurden 657g (1 mol) raffiniertes Kokosöl, 502g (1,1 mol) Glycerintricaprylat, 28g (0,3 mol) Glycerin und 1,5g Zinn(II)oxid - entsprechend 0,1 Gew.-% bezogen auf die Einsatzstoffe - 8h auf 220°C erhitzt. Es wurden nach Raffination und Desodorieren 1030g eines leicht gelb gefärbten, geruchfreien Öls erhalten, das 0,9 Gew.-% Monoglyceride, 23,1 Gew.-% Diglyceride und 76,0 Gew.-% Triglyceride enthielt.

### Beispiel 5:

657g (1 mol) raffiniertes Kokosöl, 456g (1 mol) Glycerintricaprylat 1,1g Natriummethylat - entsprechend 0,1 Gew.-% bezogen auf die Einsatzstoffe - wurden im Vakuum 6h unter Rühren auf 210°C erhitzt. Es wurden nach Raffination und Desodorieren 980g eines leicht gelb gefärbten, geruchfreien Öls erhalten.

## Patentansprüche

1. Verwendung von technischen Di-/Triglyceridgemischen, dadurch erhältlich, daß man raffinierte, überwiegend gesättigte Pflanzenöle mit (a) einer Mischung aus Glycerin und Fettsäuren der Formel **(I)**
**R**^{**1**}**COOH (I)**
bzw. deren Methylestern, oder (b) Triglyceriden auf Basis der Fettsäuren der Formel **(I)**, in der R¹CO für einen Acylrest mit 6 bis 10 Kohlenstoffatomen steht, umestert, d.h. einen Teil der längerkettigen Fettsäuren des Pflanzenöls gegen kürzerkettige Fettsäuren austauscht, als Ölkörper in kosmetischen Zubereitungen.

2. Verfahren zur Herstellung von technischen Di-/Triglyceridgemischen, bei dem man raffinierte, überwiegend gesättigte Pflanzenöle mit (a) einer Mischung aus Glycerin und Fettsäuren der Formel **(I)**
**R**^{**1**}**COOH (I)**
bzw. deren Methylestern, in der R¹CO für einen Acylrest mit 6 bis 10 Kohlenstoffatomen steht, umestert.

3. Verfahren nach Anspruch 2, **dadurch gekennzeichnet**, daß man Pflanzenöle einsetzt, die eine lodzahl im Bereich von 0,5 bis 50 aufweisen.

4. Verfahren nach den Ansprüchen 2 und 3, **dadurch gekennzeichnet**, daß man Pflanzenöle einsetzt, die ausgewählt sind aus der Gruppe, die gebildet wird von Palmöl, Palmkernöl, Babassuöl und/oder Kokosöl.

5. Verfahren nach den Ansprüchen 2 bis 4, **dadurch gekennzeichnet**, daß man Caprinsäure oder Caprinsäuremethylester einsetzt.

6. Verfahren nach den Ansprüchen 2 bis 5, **dadurch gekennzeichnet**, daß man die Pflanzenöle, die Fettsäuren bzw. Methylester und das Glycerin im molaren Verhältnis 1 : (2,5 bis 3,5): (1,0 bis 2,0) einsetzt.

## Claims

1. The use of technical di-/triglyceride mixtures obtainable by partly transesterifying, i.e. the relatively long-chain fatty acids of the vegetable oil are at least partly replaced by the relatively short-chain fatty acids of the methyl ester, refined predominantly saturated vegetable oils with (a) a mixture of glycerol and fatty acids corrsponding to formula **(I):**
**R**^{**1**}**COOH (I)**
or methyl esters thereof or (b) triglycerides based on the fatty acids corresponding to formula (I), in which R¹CO is an acyl group containing 6 to 10 carbon atoms, as oils in cosmetic formulations.

2. A process for the production of technical di-/triglyceride mixtures in which refined predominantly saturated vegetable oils are partly transesterified with a mixture of glycerol and fatty acids corresponding to formula **(I):**
**R**^{**1**}**COOH (I)**
or methyl esters thereof in which R¹CO is an acyl group containing 6 to 10 carbon atoms.

3. A process as claimed in claim 2, characterized in that vegetable oils with an iodine value of 0.5 to 50 are used.

4. A process as claimed in claims 2 and 3, characterized in that vegetable oils selected from the group consisting of palm oil, palm kernel oil, babassu oil and/or coconut oil are used.

5. A process as claimed in claims 2 to 4, characterized in that capric acid or capric acid methyl ester is used.

6. A process as claimed in claims 2 to 5, characterized in that the vegetable oils, the fatty acids or mehtyl esters and the glycerol are used in a molar ratio of 1 : (2.5 to 3.5) : (1.0 to 2.0).

## Revendications

1. Utilisation de mélanges techniques de di/triglycérides que l'on obtient en ce qu'
on transestérifie des huiles végétales raffinées, d'une manière prédominante saturées avec,
a) un mélange de glycérol et d'acides gras de formule (I),
R¹COOH (I)
ou de leurs esters méthyliques, ou
b) des triglycérides à base d'acides gras de formule (I) dans laquelle R¹CO représente un reste acyle ayant de 6 à 10 atomes de carbone, c'est-à-dire qu'on échange une partie des acides gras à chaîne plus longue de l'huile végétale contre des acides gras à chaîne plus courte, comme substances huileuses dans des préparations cosmétiques.

2. Procédé de préparation de mélanges techniques de di/triglycérides dans lequel on transestérifie des huiles végétales raffinées, d'une manière prédominante saturées, avec,
a) un mélange de glycérol et d'acides gras de formule (I),
R¹COOH (1)
ou de leurs esters méthyliques, dans laquelle R¹CO représente un reste acyle ayant de 6 à 10 atomes de carbone.

3. Procédé selon la revendication 2,
caractérisé en ce qu'
on met en oeuvre des huiles végétales qui possèdent un indice d'iode dans la zone de 0,5 à 50.

4. Procédé selon les revendications 2 et 3,
caractérisé en ce qu'
on met en oeuvre des huiles végétales qui sont choisies dans le groupe qui est formé de l'huile de palme, de l'huile de palmiste, de l'huile de babassou et/ou de l'huile de coco.

5. Procédé selon les revendications 2 à 4,
caractérisé en ce qu'
on met en oeuvre de l'acide caprique ou l'ester méthylique d'acide caprique.

6. Procédé selon les revendications 2 à 5,
caractérisé en ce qu'
on met en oeuvre les huiles végétales, les acides gras ou l'ester méthylique, et le glycérol dans le rapport molaire 1 : (2,5 à 3,5) : (1,0 à 2,0).
